Europäisches Patentamt

European Patent Office (11) Publication number: **0 144 553**

Office européen des brevets **B1**

(12) **EUROPEAN PATENT SPECIFICATION**

(45) Date of publication of patent specification: **11.11.87** (51) Int. Cl.⁴: **D 21 H 5/00, A 41 D 31/00**

(21) Application number: **84110514.1**

(22) Date of filing: **04.09.84**

(54) Non-woven activated carbon fabric.

(30) Priority: **12.09.83 US 531366**

(43) Date of publication of application:
**19.06.85 Bulletin 85/25**

(45) Publication of the grant of the patent:
**11.11.87 Bulletin 87/46**

(84) Designated Contracting States:
**AT BE CH DE FR GB IT LI NL SE**

(56) References cited:
**GB-A-1 162 110**
**US-A-3 769 144**
**US-A-3 826 712**

**ABSTRACT BULLETIN OF THE INSTITUTE OF
PAPER CHEMISTRY, vol. 51, no. 2, August
1980, page 247, no. 2159, Appleton, Wisc., US
& 7964105**

**CHEMICAL ABSTRACTS, vol. 99, no. 22, 28th
November 1983, page 105, no. 177752s,
Columbus, Ohio, US**

(73) Proprietor: **AMERICAN CYANAMID COMPANY
1937 West Main Street P.O. Box 60
Stamford Connecticut 06904-0060 (US)**

(72) Inventor: **Giglia, Robert Domenico
14 Chester Drive
Rye New York 10580 (US)**
Inventor: **Battistelli, Edward
55 Larchmont Circle
Stratford Connecticut (US)**

(74) Representative: **Wächtershäuser, Günter, Dr.
Tal 29
D-8000 München 2 (DE)**

**0 144 553**

**Description**

The invention relates to a non-woven activated carbon fabric in accordance with the preamble of the main claim. Such a fabric material is known from JP—A—64105/79.

The need for protective clothing has long been recognized by both military and commercial personnel. The manufacture and use of certain dangerous chemicals such as pesticides, toxic materials etc. in the form of sprays or mists, gases etc. requires that personnel involved therewith be provided the safest protective clothing available. Protective clothing has in the past, been manufactured from completely impermeable materials such as plastics or oil-skin. These products did not prove sufficient however, due to preventing the egress of heated air and perspiration from the wearer. Other attempts to provide protective clothing involved the use of absorbent charcoal or other materials in particulate form, however, these products also involved difficulties due to the loss of the particulate material over a period of time. The use of quilted woven carbon cloth and adhesives to bind the carbon particles, also was less than commercially successful, see U.S. Pat. Nos. 3,586,596; 3,769,144.

Accordingly, if a fabric configuration could be developed which overcame the disadvantages of previous fabrics, a step forward in the art would be achieved.

According to the present invention, a non-woven fabric material containing, as the toxic vapor absorptive ingredient therein, activated carbon particles and activated carbon fiber is produced via the wet-laying process, utilizing fibrillated acrylic fibers as the binder material, the product being air and water vapor permeable and providing the user protection from dangerous chemicals in liquid or gaseous form.

In accordance with the present invention there is provided an air and water vapor permeable, toxic vapor absorptive, non-woven fabric material comprising

    a) activated carbon fibres,
    b) activated carbon particles and
    c) fibrillated acrylic fibers.

The novel products of the present invention are prepared by wet-laying the activated carbon fibers, activated carbon particles and fibrillated acrylic fibers from a water suspension thereof. The suspension should contain from about 5—25%, by weight, based on the total weight of fibers and particles, preferably from about 10—20%, by weight, of the fibrillated acrylic fibers, from about 10—75%, by weight, same basis, preferably from about 15—65%, by weight, of the activated carbon fiber and from about 15—80%, by weight, same basis, preferably from about 25—70%, by weight, of the activated carbon particles, the total weight of the three components being 100%.

The activated carbon particles, activated carbon fiber and fibrillated acrylic fiber are wet-laid using the conventional papermaking process well known in the art. Flocculating agents and surface active agents can be incorporated into the water suspension in order to facilitate the paper making procedure as is also known in the art. The acrylic fiber may be produced from any known acrylic polymers such as polyacrylonitrile, copolymers of acrylonitrile and other copolymerizable monomers such as methyl methacrylate, ethyl acrylate etc; homopolymers and copolymers of other fiber forming monoethylenically unsaturated monomers such as vinyl acetate, vinyl chloride, styrene, vinyl pyridine acrylic esters, acrylamide and the like.

Fibrillation of the acrylic fibers, which should range from about 1 mm to about 25 mm in length, is also accomplished as is known in the art i.e. such as by high speed shearing of the fibers.

The activated carbon fibers are also well-known in the art as are methods for their production. They can be used in lengths of from about 0.3 to about 15.0 mm, preferably from 0.05 to about 10.0 mm, and can be prepared from such carbon fiber precursors as coal tar pitch, petroleum pitch, coal tar, petroleum derived thermal tar, ethylene tars, high-boiling coal tar distillates, ethylene tar distillates, gas oils or polynuclear aromatics. Also useful as precursors are polymers such as acrylonitrile homopolymers and copolymers, polyvinylalcohol, and natural and regenerated cellulose. Methods for preparing activated carbon fibers useful herein are disclosed in U.S.—A—4,069,297 and 4,285,831 which patents are hereby incorporated herein by reference.

The activated carbon powder or particles, have a particle size ranging from about 0.1 μm to about 100 μm, preferably from about 1.0 μm to about 80 μm and are also prepared from any of the carbon precursors described above.

The wet-lay sheet making process (papermaking) used herein for the production of the novel fabric material of the present invention, results in a product having unique sorptive characteristics, a thickness of at least about 0.0127 (0.005), preferably at least 0.0254 cm (0.01 inch), a high sorptive capacity to weight ratio and high porosity to fluid flow. The equilibrium loading of absorptive carbon fiber is higher than conventional activated carbon powder products. The products of the present invention are more porous than sheets containing only carbon particles. The carbon fiber, which tends to lay parallel to the plane of the sheet, produces a longer fluid flow path through the sheet which increases the time available to absorb impurities. The novel products hereof accept an unexpectedly high additional loading of active carbon powder with only a small increase in fibrillated acrylic fiber as compared to conventional products, i.e. see U.S.—A—4,088,395. The combination of active carbon fiber and active carbon particles results in a higher performance versus cost ratio than sheets which contain only one of these active ingredients.

The surface of the novel fabric material of the present invention may be embossed during or after its

2

production to improve sheet flexibility and/or porosity. The novel non-woven fabric material may be laminated to a woven, non-woven, knitted etc. backing such as matts, felts, papers, etc. produced from cotton, hemp, flax, ramie, jute, silk, wool, leather, flannel, flannelette, swansdown, poplin, cellulose ethers or esters, nylon, rayon, acetates, polythene, glass, rock wool, asbestos, in order to strengthen the material.

Lamination of the novel products hereof to the above-mentioned backing materials may be achieved by the use of water vapor and air permeable adhesives, preferably those available in the form of foams, such as rubber or acrylic latexes, polyurethanes and the like. These adhesives are self-adhering and upon curing foam and set into strong bonds.

The surface of the novel fabric material claimed herein may be rendered hydrophobic by coating with a porous silicone film or a polymer such as polytetrafluoroethylene. Additionally, a reactive coating capable of decomposing toxic agents, e.g. a coating of a sulfonated polymer to hydrolyze nerve gas, may be applied thereto so that the activated carbon particles and fibers form a second line of defence.

The fabric material of the present invention has a wide variety of uses. It is useful for protective purposes and for filtration and separation of gases. The uses include the manufacture of the fabric material into wearing apparel e.g., military uniforms, blankets, sleeping bags, bedding, surgical dressings, wrappers and containers, covers, tarpaulins, tents, curtains, gas masks, paint spraying masks, air-conditioning duct filters, flue gas deodorizers and the like.

The following examples are set forth for purposes of illustration only. All parts and percentages are by weight unless otherwise specified.

Example 1

A mixture of 14% fibrillated acrylic fibers, 18% activated carbon fiber and 68% activated carbon powder in 18 l. of water are formed into a sheet using a standard hand paper making machine. The sheet is dried under pressure at 70°C to 120°C. The resultant fabric material is then tested. The results are set forth in Table I, below.

Example 2

The procedure of Example 1 is again followed except that 12% fibrillated acrylic fiber, 59% activated carbon fiber and 29% active carbon powder are employed and the paper material is embossed after forming but before drying. The results are set forth in Table I, below.

Example 3

The procedure of Example 2 is again followed except that the fabric material is not embossed. After drying the material is laminated to a 65/35 polycotton fabric utilizing a commercially available acrylic foam adhesive. The results are set forth in Table I, below.

Example 4 (Comparative)

The procedure of Example 1 is again followed except that 45% fibrillated acrylic fiber and 55% activated carbon powder are employed. No activated carbon fibers are present. The results are set forth below in Table I.

Example 5 (Comparative)

The procedure of Example 2 is again followed except that 6.3% fibrillated acrylic fiber and 93.7% activated carbon fiber are employed. No activated carbon particles are present. The results are set forth in Table I, below.

Example 6 (Comparative)

The procedure of Example 1 is again followed except that 19.4% fibrillated acrylic fiber, 80% activated carbon fiber and 0.6% polytetrafluoroethylene are employed. No activated carbon powder is present. The results are set forth in Table I, below.

Example 7 (Comparative)

The procedure of Example 2 is employed except that 6.3% of fibrillated acrylic fibers and 93.7% of activated carbon fibers are employed. No activated carbon powder is present. Two layers of the resultant fabric material are laminated as in Example 3. The results are set forth in Table I, below.

## TABLE I

| | S | 1 | 2 | 3 | 4** | 5** | 6** | 7** |
|---|---|---|---|---|---|---|---|---|
| | | | | Example No. | | | | |
| Property weight (oz./yd$^2$) | (7.9) | (7.1) | (7.2) | (12.6) | (12.1) | (5.0) | (3.0) | (9.9) |
| g/m$^2$ | 268 | 241 | 244 | 427 | 410 | 170 | 102 | 336 |
| Thickness (inch) | (0.090) | (0.039) | (0.049) | (0.060) | (0.040) | (0.038) | (0.023) | (0.064) |
| mm | 2.29 | 0.99 | 1.24 | 1.52 | 1.02 | 0.97 | 0.58 | 1.63 |
| Air permeability (ft$^3$/min/ft$^2$) | (46) | (5) | (21.4) | (15.5) | (0.6) | (.39) | (21.1) | (93) |
| m$^3$/min/m$^2$ | 14.0 | 1.5 | 6.5 | 4.7 | 0.2 | 0.1 | 6.4 | 28.3 |
| Water vapor transmission (g/h/M$^2$) | 74 | — | 85 | 54 | — | — | — | — |
| Tensile test (lbs/in) | (36) | (18) | (18) | (65) | (44) | (2) | (6) | (2) |
| kN/m | 6.3 | 3.15 | 3.15 | 11.38 | 7.7 | 0.35 | 1.05 | 0.35 |
| Flexibility (Taber-g/cm) | 3.1 | 16.0 | 7.0 | 16.0 | 36.5 | 9.8 | 14.0 | 22.0 |
| Static CCl$_4$ absorption (mg/cm$^2$) | 14.9 | 7.5 | — | — | 15.1 | 5.6 | 2.2 | 10.5 |
| Static CCl$_4$ Sorptive capacity (% fabric wt.) | 53.6 | 30.0 | — | — | 36.9 | 31.9 | 21.9 | 30.1 |
| Dynamic CCl$_4$ absorption (mg/cm$^2$) | 2.5* | 2.0* | 3.5* | 4.3* | 6.7* | 0.85* | 0.05* | 1.35* |
| Dynamic CCl$_4$ sorptive cap. (% fabric wt.) | 9.0* | 8.0* | 14.4* | 10.1* | 16.3* | 4.8* | 0.5* | 3.9* |

*Calculated; based on 0.5 mg/l leakage point

**Comparative.

S=Standard commercial product (nylon knit backed polyurethane foam cont. activated carbon powder).

## Claims

1. An air and water vapor permeable, toxic vapor absorptive, non-woven fabric material comprising a wet-laid sheet containing acrylic fiber and activated carbon fiber characterized in that, the acrylic fibers are fibrillated and that said sheet contains activated carbon particles.

2. The fabric material of Claim 1 wherein the concentration of fibrillated acrylic fiber ranges from about 5—25% by weight, based on the total weight of fibers and particles, the concentration of activated carbon fiber ranges from about 10—75%, by weight, same basis, and the concentration of activated carbon particles ranges from about 15—80%, same basis.

3. The fabric material of Claim 1 wherein the fabric material surface is embossed.

4. The fabric material of Claim 1 wherein a backing member is adhered thereto.

5. The fabric material of Claim 3 wherein a backing member is adhered thereto.

## Patentansprüche

1. Ein für Luft und Wasserdampf permeables, toxische Dämpfe absorbierendes, nicht gewebtes Stoffmaterial, umfassend eine naß abgelegte Schicht, enthaltend acrylische Fasern und Aktivkohlefasern, dadurch gekennzeichnet, daß die acrylischen Fasern fibrilliert sind und daß Schicht Aktivkohleteilchen enthält.

2. Das Stoffmaterial gemäß Anspruch 1, wobei die Konzentration der fibrillierten acrylischen Fasern im Bereich von etwa 5 bis 25 Gew.-%, bezogen auf das Gesamtgewicht von Fasern und Teilchen, liegt, die Konzentration der Aktivkohlefasern von etwa 10 bis 75 Gew.-%, bezogen auf die gleiche Basis, beträgt und die Konzentration der Aktivkohleteilchen im Bereich von etwa 15 bis 80%, bezogen auf die gleiche Basis, liegt.

3. Das Stoffmaterial gemäß Anspruch 1, wobei die Stoffmaterialoberfläche geprägt ist.

4. Das Stoffmaterial gemäß Anspruch 1, wobei ein Verstärkungselement auf die Rückseite geklebt ist.

5. Das Stoffmaterial gemäß Anspruch 3, wobei ein Verstärkungselement auf die Rückseite geklebt ist.

**Revendications**

1. Etoffe non-tissée perméable à l'air et à la vapeur d'eau, absorbant les vapeurs toxiques, comprenant une feuille fabriquée par voie humide contenant des fibres acryliques et des fibres de carbone activé, caractérisée en ce que les fibres acryliques sont fibrillées et en ce que ladite feuille contient des particules de carbone activé.

2. Etoffe non-tissée suivant la revendication 1, caractérisée en ce que la concentration des fibres acryliques fibrillées est d'environ 5 à 25% en poids par rapport au poids total de fibres et de particules, en ce que la concentration des fibres de carbone activé est d'environ 10 à 75% en poids sur la même base, et en ce que la concentration des particules de carbone activé est d'environ 15 à 80% sur la même base.

3. Etoffe suivant la revendication 1, caractérisée en ce que la surface de l'étoffe est gaufrée.

4. Etoffe suivant la revendication 1, caractérisée en ce qu'un élément de renforcement est collé sur celle-ci.

5. Etoffe suivant la revendication 3, caractérisée en ce qu'un élément de renforcement est collé sur celle-ci.